(19) Europäisches Patentamt
European Patent Office
Office européen des brevets

(11) Veröffentlichungsnummer: **0 102 517**
**B1**

(12) # EUROPÄISCHE PATENTSCHRIFT

(45) Veröffentlichungstag der Patentschrift: **08.10.86**

(51) Int. Cl.⁴: **A 01 N 43/50,** A 01 N 43/64 // C07D405/06

(21) Anmeldenummer: **83107473.7**

(22) Anmeldetag: **29.07.83**

(54) **Fungizide Mittel.**

(30) Priorität: **10.08.82 DE 3229734**

(43) Veröffentlichungstag der Anmeldung: **14.03.84 Patentblatt 84/11**

(45) Bekanntmachung des Hinweises auf die Patenterteilung: **08.10.86 Patentblatt 86/41**

(84) Benannte Vertragsstaaten: **AT BE CH DE FR GB IT LI NL SE**

(56) Entgegenhaltungen: **EP - A - 0 035 087**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 1 (DE)**
Erfinder: **Jautelat, Manfred, Dr., Müllersbaum 28, D-5093 Burscheid (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen 1 (DE)**
Erfinder: **Reinecke, Paul, Dr., Lessingstrasse 11, D-5090 Leverkusen 3 (DE)**
Erfinder: **Scheinpflug, Hans, Dr., Am Thelenhof 15, D-5090 Leverkusen 1 (DE)**

**EP 0 102 517 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von neuen Azolyl-phenoxy-tetrahydrofuran-2-ylidenmethanen zur Bekämpfung von phytopathogenen Pilzen.

Es ist bereits bekannt geworden, dass bestimmte Azolylalkenole, wie z.B. im Phenoxyteil substituierte 1-(Imidazol-1-yl)- bzw. 1,2,4-Triazol-1-yl) -2- phenoxy -4,4- dimethyl-1-penten-3-ole, gute fungizide Eigenschaften aufweisen (vergleiche DE-A-29 28 967). Die Wirkung dieser Verbindungen ist jedoch, insbesondere bei niedrigen Aufwandmengen und -konzentrationen nicht immer ganz befriedigend.

Es wurde gefunden, dass die Azolyl-phenoxy-tetrahydrofuran -2-yliden-methane der allgemeinen Formel

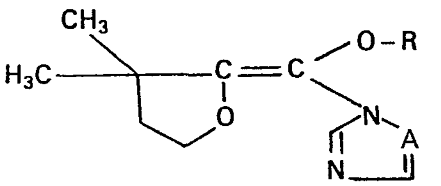 (I)

in welcher

A für ein Stickstoffatom oder die CH-Gruppe steht und

R für gegebenenfalls substituiertes Phenyl steht, sowie deren physiologisch verträgliche Säureadditions-Salze sehr gut zur Bekämpfung von phytopathogenen Pilzen geeignet sind.

Die Verbindungen der Formel (I) können in zwei geometrischen Isomerenformen vorliegen, je nach Anordnung der Gruppen, die an die Doppelbindung gebunden sind; vorzugsweise fallen sie in einem wechselnden Isomerenverhältnis an. Die vorliegende Erfindung betrifft sowohl die Verwendung der einzelnen Isomeren als auch die der Isomeren-Gemische.

Überraschenderweise zeigen die erfindungsgemäss zu verwendenden Azolyl- phenoxy- tetrahydrofuran -2- yliden-methane der Formel (I) eine bessere fungizide Wirksamkeit gegenüber phytopathogenen Pilzen als die aus dem Stand der Technik bekannten, im Phenoxyteil substituierten, 1-(Imidazol-1-yl)- bzw. (1,2,4-Triazol-1-yl) -2-phenoxy-4,4- dimethyl-1-penten-3-ole, welches chemisch und wirkungsmässig naheliegende Verbindungen sind. Die erfindungsgemässe Verwendung der neuen Stoffe stellt somit eine Bereicherung der Technik dar.

Die erfindungsgemäss zu verwendenden Azolyl-phenoxy-tetrahydrofuran -2- yliden-methane sind durch die Formel (I) allgemein definiert. In dieser Formel stehen vorzugsweise

A für ein Stickstoffatom oder die CH-Gruppe und

R für gegebenenfalls einfach bis dreifach, gleich oder verschieden substituiertes Phenyl, wobei als Substituenten vorzugsweise genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie Fluor- und Chloratomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy.

Besonders bevorzugt sind diejenigenVerbindungen der Formel (I), in denen

A für ein Stickstoffatom oder die CH-Gruppe steht und

R für gegebenenfalls einfach bis dreifach, gleich oder verschieden substituiertes Phenyl steht, wobei als Substituenten vorzugsweise genannt seien: Fluor, Chlor, Brom, Methyl, Ethyl, tert.-Butyl, Methoxy, Ethoxy, Methylthio, Trifluormethyl, Trifluormethoxy, Trifluormethylthio, Methoxycarbonyl, Ethoxycarbonyl, Nitro, Cyano, gegebenenfalls durch Chlor und/oder Methyl substituiertes Phenyl oder Phenoxy.

Bevorzugte erfindungsgemässe Verbindungen sind auch Additionsprodukte aus Säuren und denjenigen Azolyl-phenoxy- tetrahydrofuran-2-yliden-methanen der Formel (I), in denen die Substituenten A und R die Bedeutungen haben, die bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5- Naphthalindisulfonsäure.

Im einzelnen seien ausser den bei den Herstellungsbeispielen genannten Verbindungen die folgenden Verbindungen der allgemeinen Formel (I) genannt:

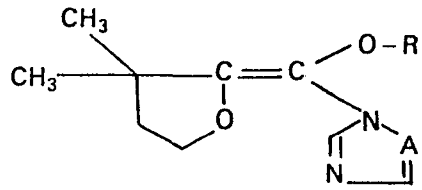

(I)

| R | A | R | A |
|---|---|---|---|
| 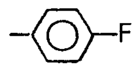 | N | 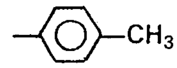 | N |
| 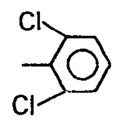 | N | 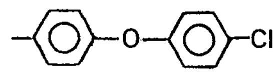 | N |
| 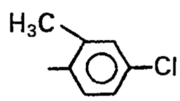 | N | 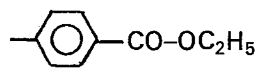 | N |
| 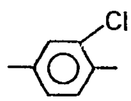 | N | 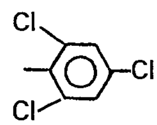 | N |
| 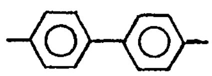 | N | 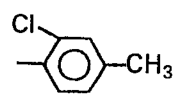 | N |
| 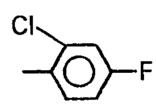 | N | 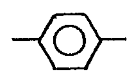 | N |
| 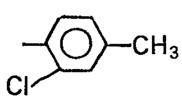 | CH | 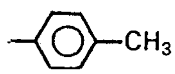 | CH |
| 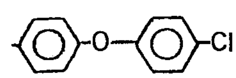 | CH | 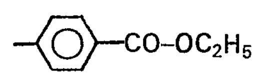 | CH |
| 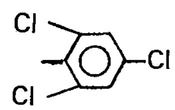 | CH | | |

Die erfindungsgemäss zu verwendenden Wirkstoffe sind in der EP-AI-0 088 874 beschrieben. Sie werden erhalten, wenn man Halogenetherketone der Formel

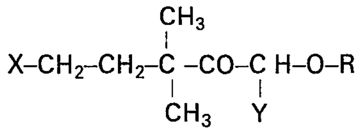

(II)

in welcher

R die oben angegebene Bedeutung hat und

X und Y für Halogen, vorzugsweise Chlor oder Brom stehen, mit Imidazol oder 1,2,4-Triazol in Gegenwart eines Verdünnungsmittels, wie beispielsweise Toluol, und in Gegenwart eines Säurebindemittels, wie beispielsweise Kaliumcarbonat, bei Temperaturen zwischen 60 und 120 °C umsetzt.

An die so erhaltenen Verbindungen der Formel (I) kann gegebenenfalls anschliessend eine Säure addiert werden.

Die Halogenetherketone der Formel (II) können nach bekannten Verfahren hergestellt werden, indem man Halogenketone der Formel

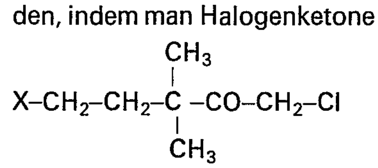

(III)

in welcher

X die oben angegebene Bedeutung hat, mit bekannten Phenolen der Formel

H–O–R (IV)

in welcher

R die oben angegebene Bedeutung hat, in üblicher Weise umsetzt und in den entstehenden Etherketonen der Formel

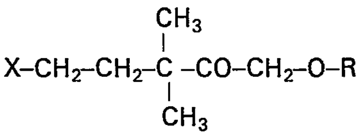

(V)

in welcher

R und X die oben angegebene Bedeutung haben das verbleibende aktive Wasserstoffatom in üblicher Weise gegen Halogen austauscht (vergleiche auch die Herstellungsbeispiele). Die Halogenetherketone der Formel (II) können gegebenenfalls ohne Isolierung direkt weiter umgesetzt werden.

Die Halogenketone der Formel (III) werden in der EP-Al-0 087 596 beschrieben.

Sie werden erhalten, indem man 2-Chlormethylen-3,3- dimethyltetrahydrofuran der Formel

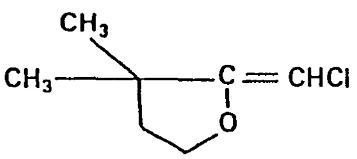

(VI)

mit aciden Verbindungen der Formel

H–X (VII)

in welcher

X die oben angegebene Bedeutung hat, gegebenenfalls in Gegenwart eines inerten organischen Lösungsmittels, wie beispielsweise Toluol oder Methylenchlorid, bei Temperaturen zwischen 20 und 150 °C umsetzt.

Das 2-Chlormethylen-3,3- dimethyltetrahydrofuran der Formel (VI) ist in der DE-A 3 204 692 beschrieben. Es wird durch sukzessive Umsetzung von 1,1,5-Trichlor-3,3- dimethyl-1-penten (vergleiche die Deutsche Offenlegungsschrift 30 29 270) mit Carboxylaten, wie beispielsweise wasserfreiem Natriumacetat, und mit Basen, wie beispielsweise Natriummethylat, in Gegenwart eines inerten organischen Lösungsmittels, wie beispielsweise Dimethylformamid, unter Rückflusstemperatur erhalten.

Die erfindungsgemäss zu verwendenden Verbindungen der Formel (I) können auch erhalten werden, wenn man

a) Halogenketone der Formel (III) mit Imidazol oder 1,2,4-Triazol gemäss den Bedingungen des eingangs erwähnten Verfahrens umsetzt (vgl. die Umsetzung von (II) mit Azolen), anschliessend die so erhaltenen Azolyltetrahydrofuran -2- yliden-methane der Formel

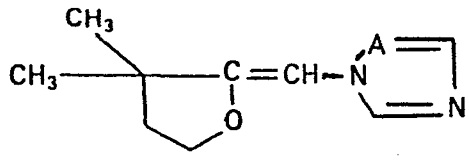

(VIII)

in welcher

A die oben angegebene Bedeutung hat, zunächst mit Halogen, insbesondere mit Brom, und danach mit Phenolen der Formel (IV) jeweils in üblicher Weise umsetzt;

b) das 2-Chlormethylen- 3,3- dimethyltetrahydrofuran der Formel (VI) in üblicher Weise mit Phenolen der Formel (IV) umsetzt, anschliessend die so erhaltenen Phenoxytetrahydrofuran-2- yliden-methane der Formel

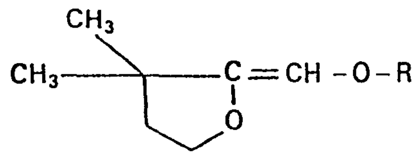

(IX)

in welcher

R die oben angegebene Bedeutung hat, zunächst in üblicher Weise mit Halogen, insbesondere mit Brom, und danach gemäss den Bedingungen des eingangs erwähnten Verfahrens mit Imidazol oder 1,2,4-Triazol umsetzt.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäss zu verwendenden Wirkstoffe weisen eine starke mikrobizide Wirkung auf und können zur Bekämpfung von unerwünschten Mikroorganismen bei Pflanzen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemässen Wirkstoffe mit besonders gutem Erfolg zur Bekämpfung von Sphaerotheca-Arten, wie gegen den Erreger des echten Gurkenmehltaus (Sphaerotheca fuliginea), von Getreidekrankheiten, wie gegen den Erreger des echten Gerstenmehltaus (Erysiphe graminis), sowie von Reiskrankheiten, wie Pyricularia oryzae und Pellicularia sasakii, eingesetzt werden.

In entsprechenden Aufwandmengen zeigen die erfindungsgemässen Wirkstoffe auch pflanzenwachstumsregulierende Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

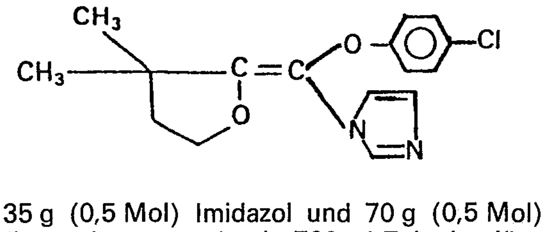

35 g (0,5 Mol) Imidazol und 70 g (0,5 Mol) Kaliumcarbonat werden in 700 ml Toluol gelöst. Diese Mischung wird bei 80 °C mit 93 g (0,26 Mol) 1- Brom -1- (4- chlorphenoxy) -5-chlor-3,3- dimethyl-2-pentanon in 200 ml Toluol versetzt. Man lässt das Reaktionsgemisch 10 Stunden bei 90 °C nachrühren, kühlt und saugt

vom anorganischen Rückstand ab. Das Filtrat wird mit Wasser gewaschen, über Natriumsulfat getrocknet und eingeengt. Der Rückstand wird säulenchromatographisch (Kieselgel: Essigester/Cyclohexan = 3/1) gereinigt. Man erhält 12,6 g (14,2% der Theorie) (4-Chlorphenoxy)-(imidazol-1-yl)-(3,3-dimethyltetrahydrofuran-2-yliden)-methan vom Schmelzpunkt 85–88 °C.

Herstellung des Ausgangsproduktes

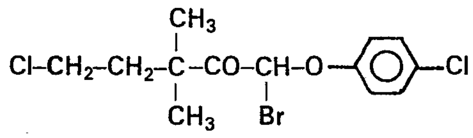

136 g (0,5 Mol) 1-(4-Chlorphenoxy)-5-chlor-3,3-dimethyl-2-pentanon werden in 1000 ml Methylenchlorid gelöst. Man lässt bei Raumtemperatur 79,9 g (1 Mol) Brom so zutropfen, dass sich die Lösung immer entfärbt. Anschliessend wird 1 Stunde bei Raumtemperatur nachgerührt und das Reaktionsgemisch durch Abdestillieren des Lösungsmittels eingeengt. Man erhält quantitativ, d.h. 177 g, 1-Brom-1-(4-chlorphenoxy)-5-chlor-3,3-dimethyl-2-pentanon, das direkt weiter umgesetzt wird.

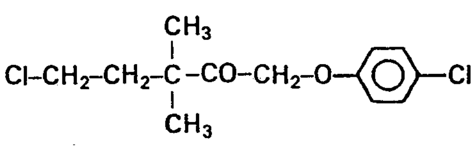

92,5 g (0,72 Mol) 4-Chlorphenol und 99,4 g (0,72 Mol) Kaliumcarbonat werden 2 Stunden in 500 ml Toluol unter Rückfluss erhitzt, wobei das Reaktionswasser azeotrop abdestilliert. Man kühlt auf 40 °C ab und versetzt mit 110 g (0,6 Mol) 1,5-Dichlor-3,3-dimethyl-2-pentanon in 300 ml Toluol. Das Reaktionsgemisch wird 5 Stunden auf 100 °C erhitzt, danach abgekühlt und vom anorganischen Rückstand abgesaugt. Das Filtrat wird mit verdünnter Natronlauge und Wasser gewaschen, über Natriumsulfat getrocknet und eingeengt. Man erhält 136,3 g (82,6% der Theorie) rohes 1-(4-Chlorphenoxy)-5-chlor-3,3-dimethyl-2-pentanon, das direkt weiter umgesetzt wird.

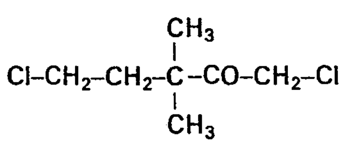

In 476 g (3,25 Mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert und die Innentemperatur steigt bis auf 30 °C. Nach vollständiger Sättigung mit Chlorwasserstoff wird das Reaktionsgemisch 2 Stunden bei Raumtemperatur nachgerührt. Überschüssiger Chlorwasserstoff wird zunächst in die Wasserstrahlpumpe gezogen, dann wird bei gutem Vakuum destilliert. Man erhält 531 g (90% der Theorie) 1,5-Dichlor-3,3-dimethyl-2-pentanon vom Schmelzpunkt 85–90 °C/0,3 mbar.

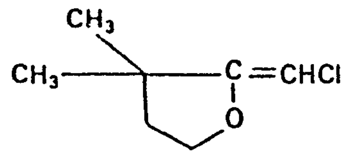

806 g (4 Mol) 1,1,5-Trichlor-3,3-dimethyl-1-penten werden mit 360 g (4,4 Mol) wasserfreiem Natriumacetat in 1 l Dimethylformamid 6 Stunden unter Rückfluss erhitzt. Nach Abkühlung auf ca. 100 °C tropft man 1,6 l (8 Mol) 30%-ige Natriummethylatlösung in Methanol ein und erhitzt weitere 4 Stunden unter Rückfluss. Die kalte Lösung wird in Wasser gegossen und mit Methylenchlorid mehrfach extrahiert.

Nach Trocknen der Lösung und Abdestillieren des Lösungsmittels bleiben 654 g Produkt zurück, das über eine Kolonne fraktioniert wird. Man erhält 522 g (89% der Theorie) 2-Chlormethylen-3,3-dimethyltetrahydrofuran vom Siedepunkt 84–87 °C/20 mbar.

Analog werden die folgenden Verbindungen der allgemeinen Formel (I)

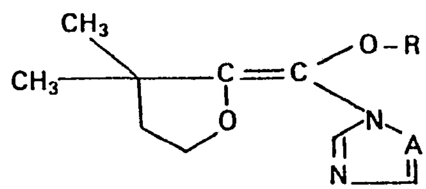

(I)

erhalten:

| Bsp. Nr. | R | A | Schmelzpunkt (°C) bzw. $n_D^{20}$ |
|---|---|---|---|
| 2 | 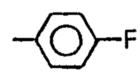 | CH | 214 |
| 3 | 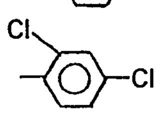 | CH | 119 (A-Form) |
| 4 | 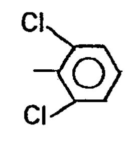 | CH | 82 |
| 5 | 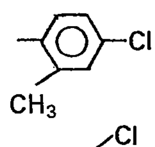 | CH | 109 (A-Form) |
| 6 | 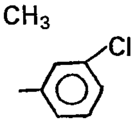 | CH | 1,5607 |
| 7 | 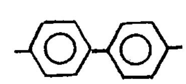 | CH | 95–105 |

| Bsp. Nr. | R | A | Schmelzpunkt (°C) bzw. $n_D^{20}$ |
|---|---|---|---|
| 8 | 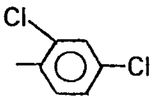 | CH | 95–105 |
| 9 | 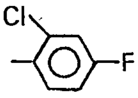 | CH | Kristall-masse |
| 10 | 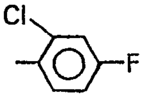 | CH | 118 (A-Form) |
| 11 | 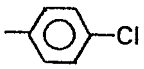 | N | 124 |
| 12 | 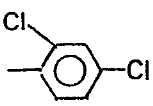 | N | zähes Öl |
| 13 | 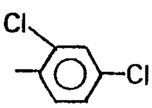 | N | 115–20 (A-Form) |
| 14 | 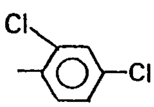 | N | 1,5578 (B-Form) |
| 15 | 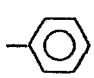 | CH | 85–90 |
| 16 | 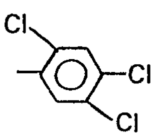 | N | 104 |

A- und B-Form: die beiden möglichen Isomerenformen

Verwendungsbeispiele

In den nachfolgenden Beispielen werden die nachstehend angegebenen Verbindungen als Vergleichssubstanzen eingesetzt:

(A) 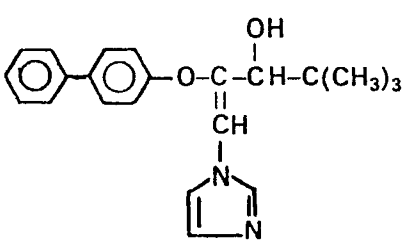

(B) 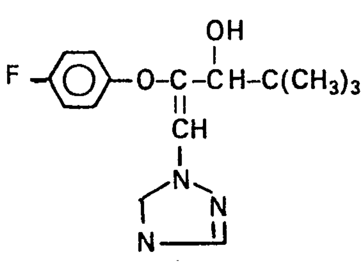

(C) 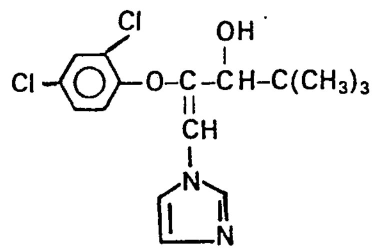

(D) 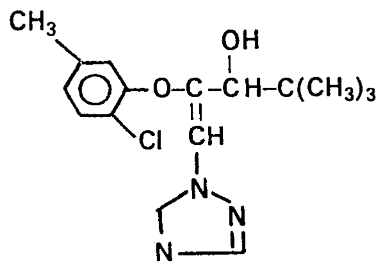

(E) 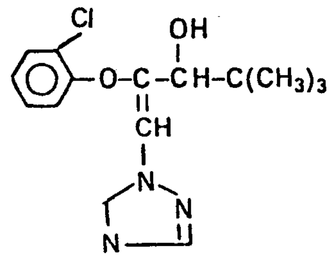

(F) 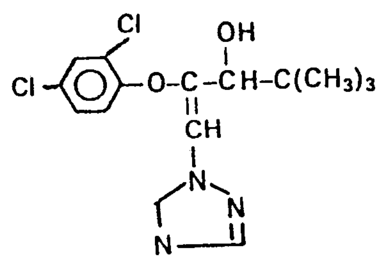

Beispiel A

Sphaerotheca-Test (Gurke) / protektiv

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschliessend bei 23 bis 24 °C und bei einer relativen Luftfeuchtigkeit von ca. 75% im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 11, 1, 2, 3, 8, 4, 5, 6, 12, 9, 10 und 15.

Tabelle A
Sphaerotheca-Test (Gurke) / protektiv

| Wirkstoff | Befall in % bei einer Wirkstoffkonzentration von 0,0005% |
|---|---|
| 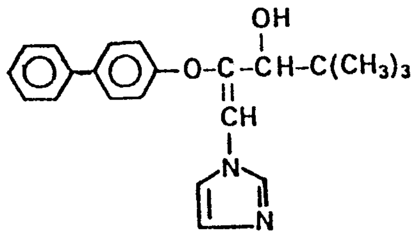 (bekannt) (A) | 62 |
| 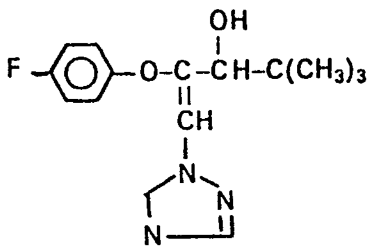 (bekannt) (B) | 91 |
| 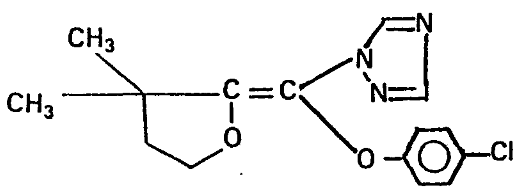 (11) | 12 |
| 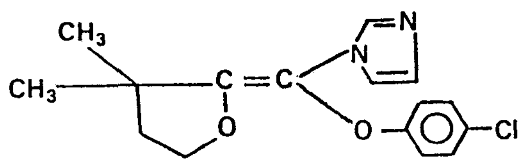 (1) | 12 |
| 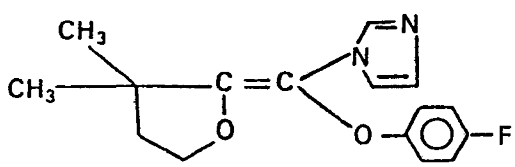 (2) | 25 |
| 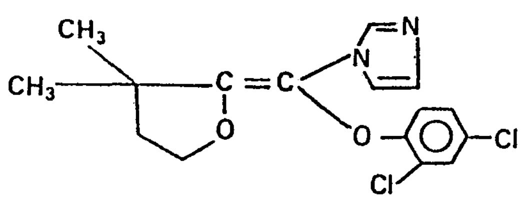 (3) | 10 |

| Wirkstoff | Befall in % bei einer Wirkstoffkonzentration von 0,0005% |
|---|---|
| 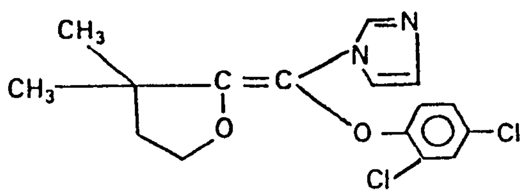 (8) | 10 |
| 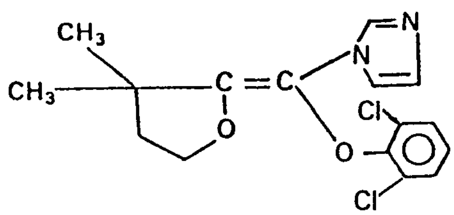 (4) | 10 |
| 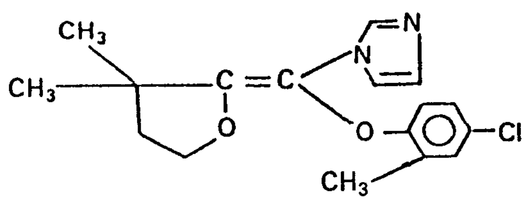 (5) | 10 |
| 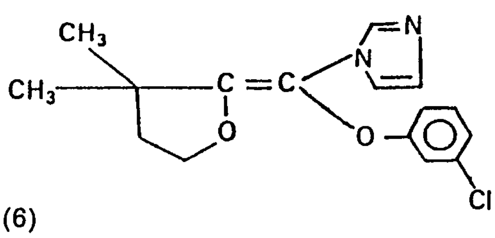 (6) | 50 |
| 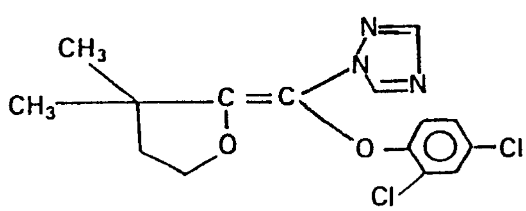 (12) | 0 |
| 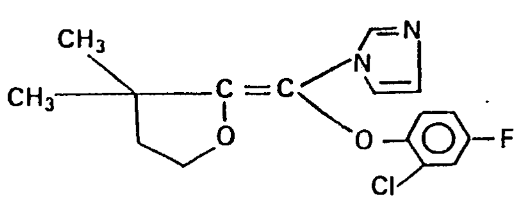 (9) | 0 |

| Wirkstoff | Befall in % bei einer Wirkstoffkonzentration von 0,0005% |
|---|---|
| 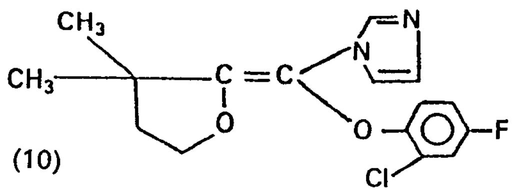 (10) A–Form | 5 |
| 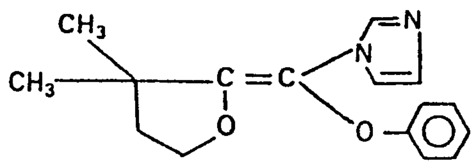 (15) | 20 |

Beispiel B

Erysiphe-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 11, 1, 2, 3, 4, 5, 8, und 6.

Tabelle B

Erysiphe-Test (Gerste)/protektiv

| Wirkstoff | Wirkstoffkonzentration in der Spritzbrühe in Gew.-% | Krankheitsbefall in % der unbehandelten Kontrolle |
|---|---|---|
| (C) 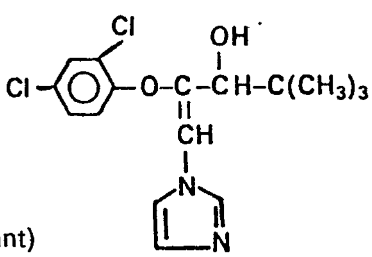 (bekannt) | 0,0025 | 100 |
| (D) 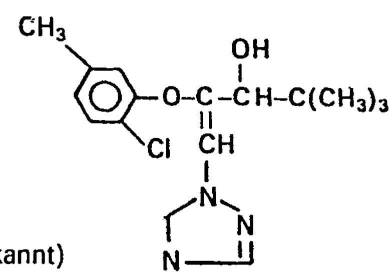 (bekannt) | 0,0025 | 83,8 |

| Wirkstoff | Wirkstoffkonzentration in der Spritzbrühe in Gew.-% | Krankheitsbefall in % der unbehandelten Kontrolle |
|---|---|---|
| 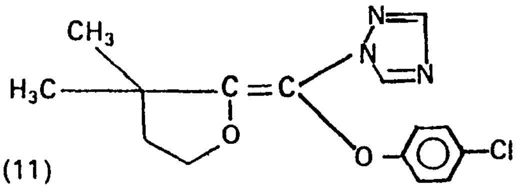 (11) | 0,0025 | 0,0 |
| 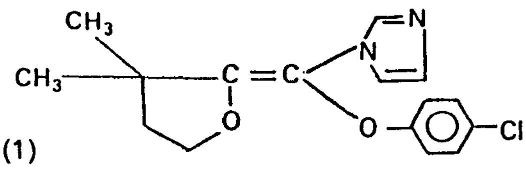 (1) | 0,0025 | 8,8 |
| 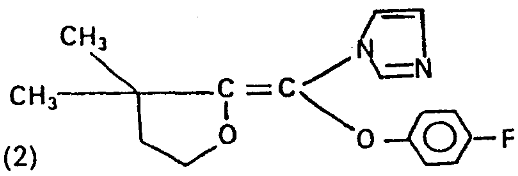 (2) | 0,0025 | 12,5 |
| 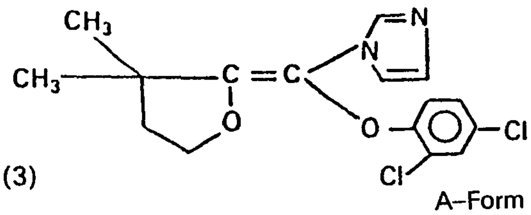 (3) A–Form | 0,0025 | 8,8 |
| 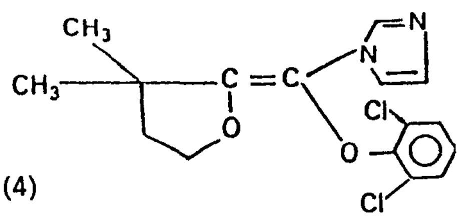 (4) | 0,0025 | 12,5 |
| 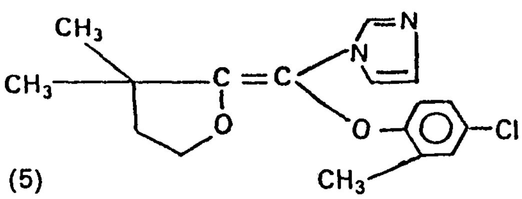 (5) A–Form | 0,0025 | 0,0 |
| 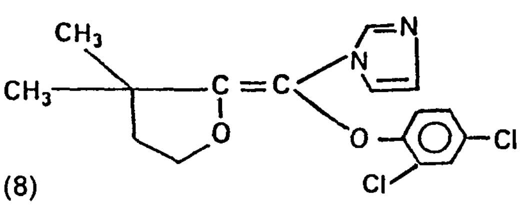 (8) | 0,0025 | 8,8 |
| 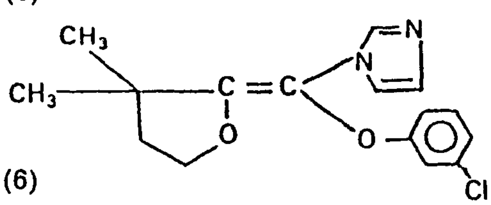 (6) | 0,0025 | 3,8 |

Beispiel C

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäss folgender Herstellungsbeispiele: 2, 3, 4, 6, 7 und 10.

Tabelle C

Pyricularia-Test (Reis)/protektiv

| Wirkstoffe | Wirkstoffkonzentration in % | Krankheitsbefall in % der unbehandelten Kontrolle |
|---|---|---|
| 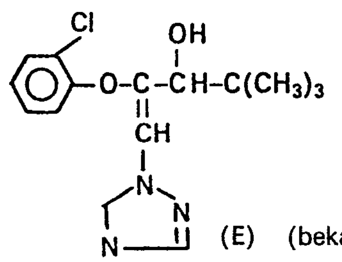 (E) (bekannt) | 0,025 | 100 |
| 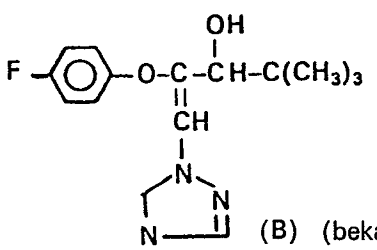 (B) (bekannt) | 0,025 | 100 |
| 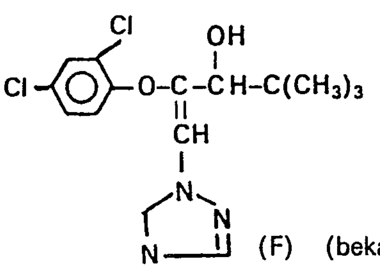 (F) (bekannt) | 0,025 | 100 |
| 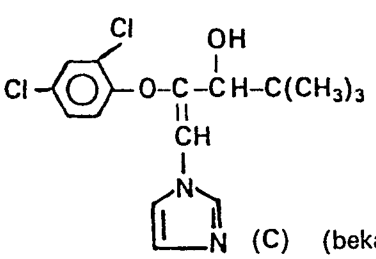 (C) (bekannt) | 0,025 | 100 |

| Wirkstoffe | Wirkstoffkonzentration in % | Krankheitsbefall in % der unbehandelten Kontrolle |
|---|---|---|
| 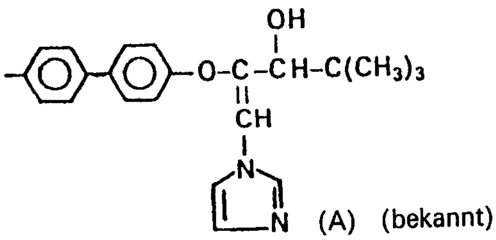 (A) (bekannt) | 0,025 | 100 |
| 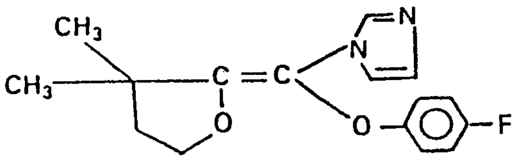 (2) | 0,025 | 13 |
| 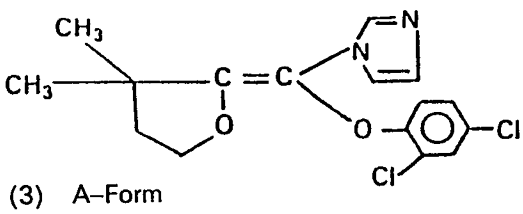 (3) A–Form | 0,025 | 0 |
| 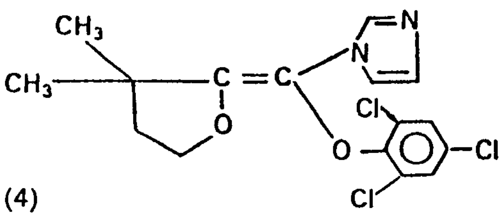 (4) | 0,025 | 25 |
| 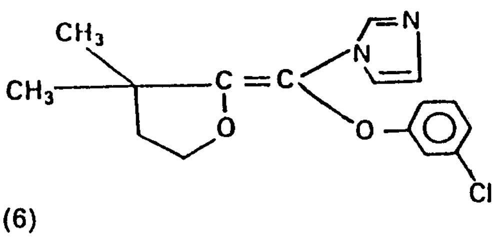 (6) | 0,025 | 0 |
| 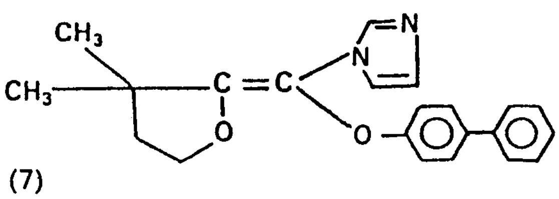 (7) | 0,025 | 25 |
| 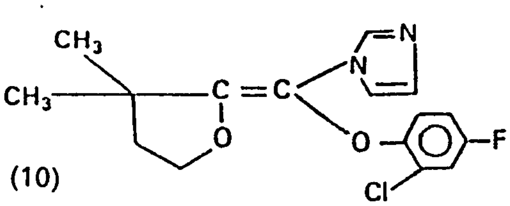 (10) A–Form | 0,025 | 0 |

Beispiel D
Pellicularia-Test (Reis)
Lösungsmittel: 12,5 Gewichtsteile Aceton
Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnass gespritzt. Die Pflanzen verbleiben bis zum Abtrocknen im Gewächshaus. Anschliessend werden die Pflanzen mit Pellicularia sasakii inokuliert und bei 25 °C und 100% relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäss folgender Herstellungsbeispiele: 11, 2, 3, 4, 5, 6, 12 und 10.

Tabelle D
Pellicularia-Test (Reis)

| Wirkstoff | Wirkstoffkonzentration in % | Krankheitsbefall in % der unbehandelten Kontrolle |
|---|---|---|
| 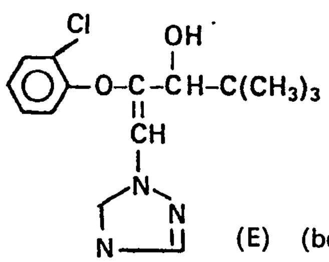 (E) (bekannt) | 0,025 | 100 |
| 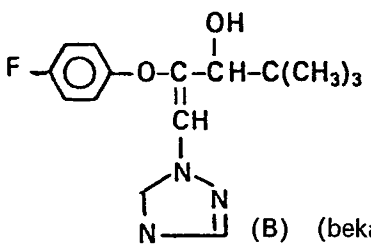 (B) (bekannt) | 0,025 | 50 |
| 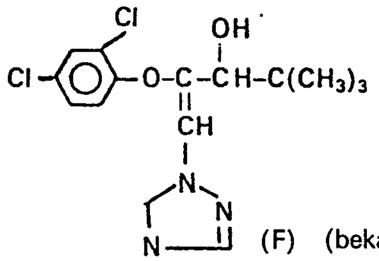 (F) (bekannt) | 0,025 | 100 |
| 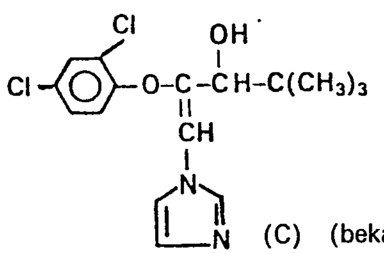 (C) (bekannt) | 0,025 | 100 |

| Wirkstoff | Wirkstoffkonzentration in % | Krankheitsbefall in % der unbehandelten Kontrolle |
|---|---|---|
| 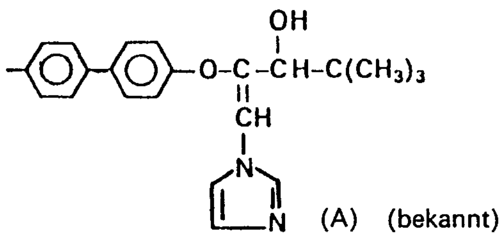 (A) (bekannt) | 0,025 | 75 |
| 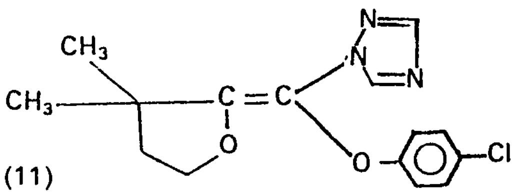 (11) | 0,025 | 13 |
| 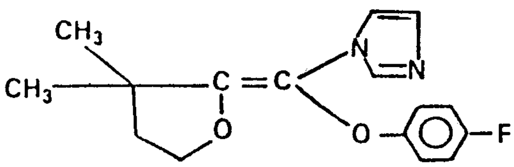 (2) | 0,025 | 13 |
| 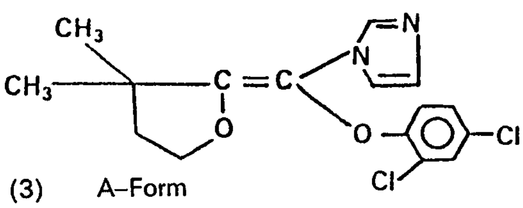 (3) A–Form | 0,025 | 0 |
| 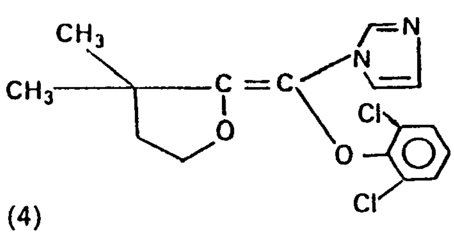 (4) | 0,025 | 13 |
| 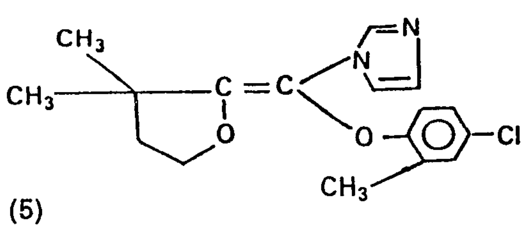 (5) | 0,025 | 25 |

| Wirkstoff | Wirkstoffkonzentration in % | Krankheitsbefall in % der unbehandelten Kontrolle |
|---|---|---|
| 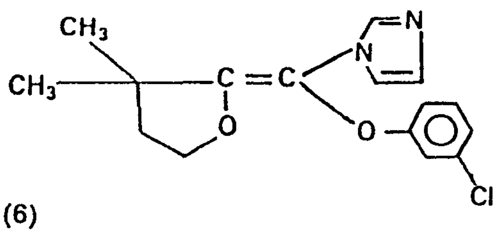 (6) | 0,025 | 13 |
| 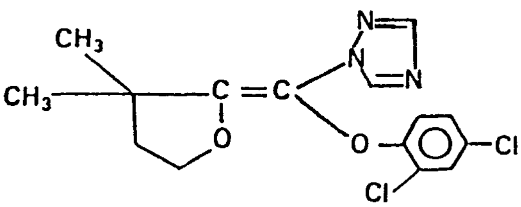 (12) | 0,025 | 0 |
| 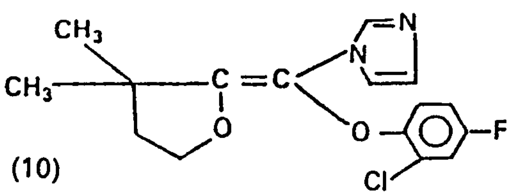 (10) A-Form | 0,025 | 0 |

**Patentansprüche**

1. Verfahren zur Bekämpfung von phytopathogenen Pilzen, dadurch gekennzeichnet, dass man Azolyl- phenoxy-tetrahydrofuran- 2- yliden-methane der allgemeinen Formel (I)

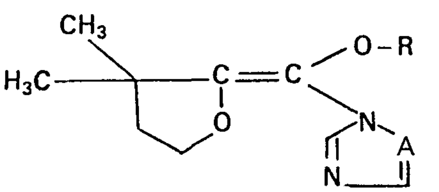

(I)

in welcher

A für ein Stickstoffatom oder die CH-Gruppe steht und

R für gegebenenfalls substituiertes Phenyl steht,

oder deren physiologisch verträgliche Säureadditionssalze auf Pilze oder ihren Lebensraum einwirken lässt.

2. Verfahren gemäss Anspruch 1, wobei in Formel (I)

A für ein Stickstoffatom oder die CH-Gruppe steht und

R für Phenyl steht, welches gegebenenfalls ein- bis dreifach, gleich oder verschieden substituiert sein kann durch Halogen, Alkyl mit 1 bis 4 Kohlenstoffatomen, Alkoxy und Alkylthio und jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie Fluor- und Chloratomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl oder Phenoxy.

3. Verfahren gemäss Anspruch 1, wobei in Formel (I)

A für ein Stickstoffatom oder die CH-Gruppe steht und

R für Phenyl steht, welches gegebenenfalls einfach bis dreifach, gleich oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Methoxycarbonyl, Ethoxycarbonyl, Nitro, Cyano und schliesslich durch gegebenenfalls durch Chlor und/oder Methyl substituiertes Phenyl oder Phenoxy.

4. Verwendung von Azolyl-phenoxy-tetrahydrofuran- 2-yliden-methanen der Formel (I) in Anspruch 1 als Pflanzenschutzmittel.

5. Verwendung von Azolyl-phenoxy-tetrahydrofuran- 2-yliden-methanen der Formel (I) in

Anspruch 1 zur Bekämpfung von phytopathogenen Pilzen.

**Revendications**

1. Procédé en vue de combattre les champignons phytopathogènes, caractérisé en ce qu'on fait agir, sur les champignons ou leur biotope, des azolyl-phénoxy-tétrahydrofuran -2- ylidène-méthanes de formule générale (I):

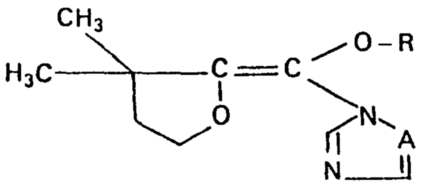

(I)

dans laquelle

A représente un atome d'azote ou le groupe CH, et

R représente un groupe phényle éventuellement substitué, ou leurs sels d'addition d'acide physiologiquement acceptables.

2. Procédé selon la revendication 1, dans lequel, dans la formule (I):

A représente un atome d'azote ou le groupe CH, et

R représente le groupe phényle qui peut éventuellement être substitué une à trois fois de manière identique ou différente par un atome d'halogène, un groupe alkyle contenant 1 à 4 atomes de carbone, un groupe alcoxy et un groupe alkylthio contenant chacun 1 ou 2 atomes de carbone, un groupe halogénalkyle, un groupe halogénalcoxy et un groupe halogénalkylthio contenant chacun 1 ou 2 atomes de carbone et 1 à 5 atomes d'halogènes identiques ou différents tels que des atomes de fluor et de chlore, un groupe alcoxycarbonyle contenant 1 à 4 atomes de carbone dans la fraction alkyle, un groupe nitro, un groupe cyano ou un groupe phénoxy ou un groupe phényle éventuellement substitué par un atome d'halogène et/ou par un groupe alkyle contenant 1 ou 2 atomes de carbone.

3. Procédé selon la revendication 1, dans lequel, dans la formule (I):

A représente un atome d'azote ou le groupe CH, et

R représente le groupe phényle qui peut éventuellement être substitué une à trois fois de manière identique ou différente par l'atome de fluor, l'atome de chlore, l'atome de brome, le groupe méthyle, le groupe éthyle, le groupe tert-butyle, le groupe méthoxy, le groupe éthoxy, le groupe méthylthio, le groupe trifluorométhyle, le groupe trifluorométhoxy, le groupe trifluorométhylthio, le groupe méthoxycarbonyle, le groupe éthoxycarbonyle, le groupe nitro, le groupe cyano et, enfin, par le groupe phénoxy ou le groupe phényle éventuellement substitué par l'atome de chlore et/ou le groupe méthyle.

4. Utilisation d'azolyl-phénoxy- tétrahydrofuran-2- ylidène-méthanes de formule (I) selon la revendication 1 comme agents de protection des plantes.

5. Utilisation d'azolyl-phénoxy- tétrahydrofuran- 2-ylidène-méthanes de formule (I) selon la revendication 1 pour combattre les champignons phytopathogènes.

**Claims**

1. Method of combating phytopathogenic fungi, characterised in that azolyl-phenoxy-tetrahydrofuran-2- ylidene-methanes of the general formula (I)

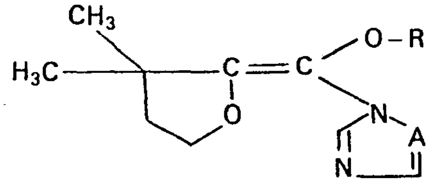

(I)

in which

A represents a nitrogen atom or the CH group and

R represents optionally substituted phenyl, or their physiologically tolerated acid addition salts are allowed to act on fungi or their environment.

2. Process according to Claim 1, wherein, in formula (I)

A represents a nitrogen atom or the CH group and

R represents phenyl which is optionally mono- to trisubstituted by identical or different substituents from the group comprising halogen, alkyl with 1 to 4 carbon atoms, alkoxy and alkylthio with in each case 1 to 2 carbon atoms, halogenoalkyl, halogenoalkoxy and halogenoalkylthio with in each case 1 to 2 carbon and 1 to 5 identical or different halogen atoms, such as fluorine and chlorine atoms, alkoxycarbonyl with 1 to 4 carbon atoms in the alkyl part, nitro, cyano or phenyl or phenoxy which are optionally substituted by halogen and/or alkyl with 1 to 2 carbon atoms.

3. Process according to Claim 1, wherein, in formula (I)

A represents a nitrogen atom or the CH group and

R represents phenyl which can be optionally mono- to tri-substituted by identical or different substituents from the group comprising fluorine, chlorine, bromine, methyl, ethyl, tert.-butyl, methoxy, ethoxy, methylthio, trifluoromethyl, trifluoromethoxy, trifluoromethylthio, methoxycarbonyl, ethoxycarbonyl, nitro, cyano and finally by phenyl or phenoxy which are optionally substituted by chlorine and/or methyl.

4. Use of azolyl-phenoxy-tetrahydrofuran-2-ylidene-methanes of the formula (I) in Claim 1 as plant protection agents.

5. Use of azolyl-phenoxy-tetrahydrofuran -2-ylidene-methanes of the formula (I) in Claim 1 for combating phytopathogenic fungi.